(19) Europäisches Patentamt
European Patent Office
Office européen des brevets

(11) Veröffentlichungsnummer: **0 063 299**
**A1**

# (12) EUROPÄISCHE PATENTANMELDUNG

(21) Anmeldenummer: **82102885.9**

(22) Anmeldetag: **05.04.82**

(51) Int. Cl.³: **C 07 D 263/44**
C 07 D 263/52

(30) Priorität: **18.04.81 DE 3115649**

(43) Veröffentlichungstag der Anmeldung:
**27.10.82 Patentblatt 82/43**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Fauss, Rudolf, Dr.**
**Gerstenkamp 10**
**D-5000 Köln 80(DE)**

(72) Erfinder: **Schäfer, Walter, Dr.**
**Wolfskaul 6**
**D-5000 Köln 80(DE)**

(72) Erfinder: **Findeisen, Kurt, Dr.**
**In der Follmuehle 10**
**D-5068 Odenthal(DE)**

(54) **Verfahren zur Herstellung von N-Aryloxazolidin-2,4-dionen.**

(57) Die Erfindung betrifft ein Verfahren zur Herstellung von N-Aryloxazolidin-2,4-dionen der Formel (I),

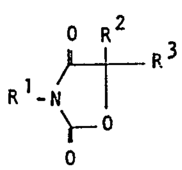

(1)

bei dem geeignete 2-Hydroxycarbonsäureester mit Harnstoffen und/oder Ureten und geeigneten primären aromatischen Aminen umgesetzt werden.

EP 0 063 299 A1

Croydon Printing Company Ltd.

0063299

BAYER AKTIENGESELLSCHAFT 5090 Leverkusen, Bayerwerk

Zentralbereich
Patente, Marken und Lizenzen Bas/bo/c
(IVa)

Verfahren zur Herstellung von N-Aryloxazolidin-2,4-dionen

Oxazolidin-2,5-dione sind bekannt. Sie können durch Umsetzung von 2-Hydroxycarbonsäureestern mit Isocyanaten oder mit substituierten Harnstoffen hergestellt werden (DE-OS 1 811 843, 2 022 494, 2 207 576 und 2 827 414).

Die bei diesen Verfahren eingesetzten Isocyanate und substituierten Harnstoffe werden aus entsprechenden Aminkomponenten und Phosgen hergestellt, was aufwendig ist und die Wirtschaftlichkeit der Verfahren beeinträchtigt (DE-OS 1 811 843 und DE-OS 2 827 414).

Gegenstand der vorliegenden Erfindung ist ein Verfahren zur Herstellung von N-Aryloxazolidin-2,4-dionen der Formel I,

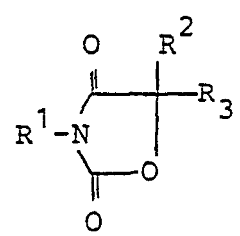

(I)

in der

$R^1$ für Aryl steht,

$R^2$ und $R^3$ unabhängig voneinander für Wasserstoff, gegebenenfalls substituiertes Alkyl, Cycloalkyl, Vinyl stehen oder gemeinsam mit dem Kohlenstoffatom an das sie gebunden sind, einen gegebenenfalls substituierten Cycloalkylring bilden,

das dadurch gekennzeichnet ist, daß 2-Hydroxycarbonsäureester der Formel II,

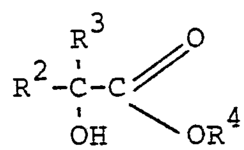

(II)

in der

$R^2$ und $R^3$ die oben angegebene Bedeutung haben und

$R^4$ für Alkyl oder Cycloalkyl steht,

mit Harnstoff und/oder Ureten der Formel III,

$$H(NH\underset{\underset{O}{\|}}{C})_nNH_2 \qquad \text{(III)}$$

in der

n  für eine ganze Zahl von 1 bis 4 steht,

und primären aromatischen Aminen der Formel IV,

$$R^1\text{-}NH_2 \qquad (IV)$$

worin

$R^1$ die oben angegebene Bedeutung hat,

bei Temperaturen von 100-250°C umgesetzt werden.

Der wesentliche Vorteil des erfindungsgemäßen Verfahrens ist darin zu sehen, daß Ausgangsprodukte eingesetzt werden können, die ohne Verwendung von Phosgen hergestellt werden können (EP-A 0018581, 0018583 und 0000563).

Verwendet man bei dem Verfahren beispielsweise Milchsäureethylester, 3,4-Dichloranilin und Harnstoff, so kann der Reaktionsablauf durch das folgende Formelschema wiedergegeben werden:

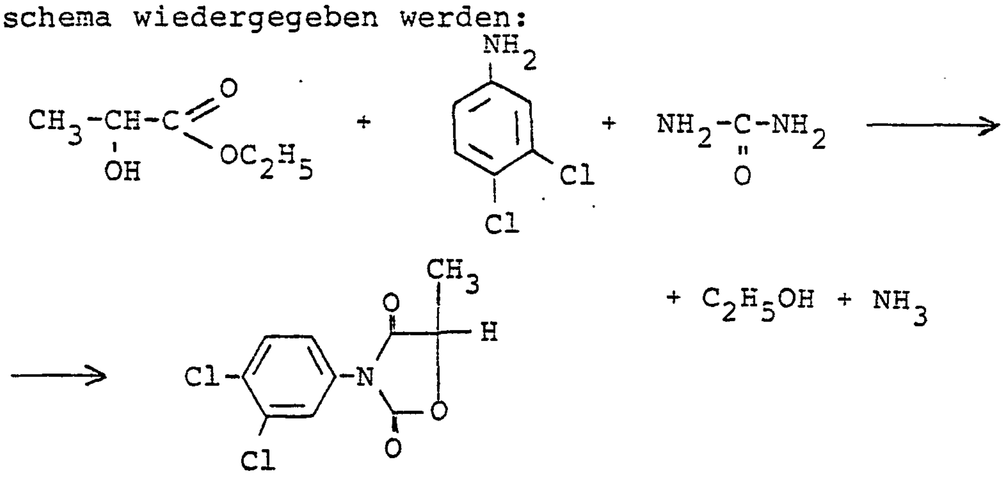

Die Ausgangsstoffe der Formel II sind durch die Formel II allgemein definiert. In Formel II stehen

$R^2$ und $R^3$ unabhängig voneinander vorzugsweise für Wasserstoff, gegebenenfalls substituiertes $C_1$-$C_6$-Alkyl, $C_3$-$C_6$-Cycloalkyl, $C_2$-$C_6$-Vinyl, wobei $R^2$ und $R^3$ auch gemeinsam mit dem C-Atom an das sie gebunden sind einen $C_3$-$C_6$-Cycloalkylring bilden können,

$R^4$ vorzugsweise für $C_1$-$C_{10}$-Alkyl und $C_3$-$C_6$-Cycloalkyl.

Besonders bevorzugt sind Verbindungen der Formel II in welcher

$R^2$ und $R^3$ unabhängig voneinander für Wasserstoff, gegebenenfalls substituiertes Methyl, Ethyl, Propyl, Cyclopropyl, Cyclopentyl, Cyclohexyl, Vinyl stehen, wobei $R^2$ und $R^3$ auch gemeinsam mit dem C-Atom an das sie gebunden sind, einen Cyclopropylring bilden können und

$R^4$ für $C_1$-$C_4$-Alkyl steht.

$R^2$ und $R^3$ können mit Halogen wie Fluor, Chlor und Brom und $C_1$-$C_4$-Alkyl substituiert sein.

Neben den in den Herstellungsbeispielen genannten Verbindungen der Formel II seien bevorzugt genannt:

α-Hydroxyessigsäure-, α-Hydroxypropionsäure-, α-Hydroxybuttersäure-, α-Hydroxyisobuttersäure-, α-Hydroxy-α-methylpropionsäure-, α-Hydroxy-α-methyl-buttersäure-, α-Hydroxy-α-methyl-capronsäure-, α-Hydroxy-α-methyl-α-chlor-propionsäure-, α-Hydroxycyclohexancarbonsäure-, α-Hydroxy-cyclopropancarbonsäure-, α-Hydroxy-2-methyl-cyclopropancarbonsäure-, α-Hydroxy-α-methyl-α-vinyl-essigsäure-, α-Hydroxy-cyclopentan-carbonsäure-, α-Hydroxy-2,2-dichlor-cyclopropancarbonsäure-, α-Hydroxy-3-methyl,2,2-dichlor-cyclopropancarbonsäuremethyl-, -ethyl-, -propyl- oder -butyl-ester.

Die Ausgangsstoffe der Formel III sind durch die Formel III allgemein definiert. In Formel III steht der Index n vorzugsweise für 1 oder 2. Besonders erwähnt seien Harnstoff und Biuret.

Die Ausgangsstoffe der Formel IV sind durch Formel IV allgemein definiert. In Formel IV steht

$R^1$ vorzugsweise für $C_6$-$C_{10}$-Aryl. Besonders erwähnt sei gegebenenfalls substituiertes Phenyl und Naphthyl.

Besonders bevorzugt sind Verbindungen der Formel IV, in welcher

$R^1$ für Phenyl, das gegebenenfalls mit Halogen wie Fluor, Chlor und Brom, Nitrogruppen, $C_1$-$C_4$-Alkyl und -Alkoxy, Phenoxy und Phenyl, substituiert sein kann, steht.

Neben den in den Herstellungsbeispielen genannten Verbindungen der Formel IV seien bevorzugt genannt: Anilin, Naphthylamin, 2-, 3- und 4-Fluorphenylamin, 2-, 3- und 4-Chlorphenylamin, 2-, 3- und 4-Bromphenylamin-, 2,3-Dichlorphenylamin, 2,3,6-Trichlorphenylamin, 2-, 3- und 4-Methoxyphenylamin, 2-, 3- und 4-Methylphenylamin, 3,4-Dichlorphenylamin, 2,6-Dichlorphenylamin, 2,3,4-Trichlorphenylamin, 3,4,5-Trichlorphenylamin, 2,4,6-Trichlorphenylamin, 2,3,5-Trichlorphenylamin. Bevorzugt hervorgehoben sei 3,5-Dichlorphenylamin.

Die als Ausgangsstoffe verwendeten 2-Hydroxycarbonsäureester der Formel II, Harnstoff und Urete der Formel III und die Amine der Formel IV sind bekannte Verbindungen.

Das erfindungsgemäße Verfahren wird im allgemeinen ohne Verdünnungsmittel durchgeführt. In besonderen Fällen können jedoch unter den Reaktionsbedingungen inerte Verdünnungsmittel mit einem Siedepunkt, der im Temperaturbereich der Umsetzung liegt, verwendet werden. Folgende Verdünnungsmittel seien genannt:

n-Nonan, n-Butylcyclohexan, Decahydronaphthalin, n-Undecan, n-Dodecan, n-Hexylcyclohexan, Dipenten, 1-Dodecen, Isopropylbenzol, 1,3-Diethylbenzol, Inden, n-Butylbenzol, Tetralin, Chlorbenzol, 4-Chlortoluol, 1,2-Dichlorbenzol, 2,4-Dichlortoluol, 1,2,4-Trichlorbenzol, 2-Chlor-4-isopropyl-1-methylbenzol, Anisol, Cyclohexylethylether, Diethylenglykoldimethylether, Benzylmethylether, 4-Methoxytoluol, Parachloranisol, Di-n-hexylether, Phenyl-n-propylketon, Benzophenon, Acetophenon, Formamid, N,N-Dimethylformamid, N,N-Diethylformamid, N-Methylformamid, Dimethylacetamid, N-Methylpyrrolidon, Caprolactam, Phenol, substituierte Phenole, Sulfolan, Hexamethylphosphorsäuretriamid, Dimethylsulfoxid, Ethylenglykolmonomethyletheracetat, Di-n-propylcarbonat, Cyclohexylacetat, Diisobutylcarbonat, Diethylenglykolmonomethyletheracetat, Diisoamylcarbonat, 2-Ethylpyridin, N,N-Dimethyl-2-methylanilin, N,N-Dimethylanilin, N-Methyl-N-ethylanilin, N,N-Dimethyl-2-chloranilin, N,N-Diethylanilin, Chinolin, Nitrocyclohexan, Nitrobenzol, 2-Nitrotoluol, 2,4-Dimethyl-1-nitrobenzol, Acetonitril, N-Capronitril, Benzonitril, Tolunitril, Phenylacetonitril, Essigsäureethylester, Toluol, Diethylenglykoldienethylether und Tetramethylharnstoff.

Die erfindungsgemäße Umsetzung wird im Temperaturbereich von 100-250°C, bevorzugt im Bereich von 130-240°C ausgeführt.

Die erfindungsgemäße Umsetzung wird bei Normaldruck ausgeführt. Es kann jedoch in speziellen Fällen von Vorteil sein, gegen Ende der Reaktion einen geringen Unterdruck bis zu 20 mbar anzuwenden.

Im erfindungsgemäßen Verfahren werden vorteilhaft stöchiometrische Mengen der Ausgangsverbindungen eingesetzt. Überschüsse an Reaktionskomponenten können in speziellen Fällen durchaus vorteilhaft sein.

Der bei der Umsetzung verwendete Harnstoff und/oder die Urete können als Mischungen eingesetzt werden. Besonders vorteilhaft kann der bei der Harnstoffherstellung anfallende biurethaltige Harnstoff eingesetzt werden.

Das erfindungsgemäße Verfahren kann kontinuierlich und diskontinuierlich ausgeführt werden.

Das erfindungsgemäße Verfahren wird im allgemeinen wie folgt durchgeführt: 2-Hydroxycarbonsäureester und die Umsetzungspartner Harnstoff und/oder Urete und primäres aromatisches Amin werden auf die Umsetzungstemperatur gebracht. Während der Dauer der Umsetzung kann der dabei entstehende Alkohol, sofern sein Siedepunkt unterhalb der Umsetzungstemperatur liegt, abdestilliert werden. Nach Beendigung der Umsetzung und Abkühlung auf Raumtemperatur können die entstandenen N-Aryloxazolidin-2,4-dione auf übliche Weise isoliert und beispielsweise durch Kristallisation oder Destillation gereinigt werden.

Die im erfindungsgemäßen Verfahren eingesetzten 2-Hydroxycarbonsäureester besitzen ein asymmetrisches C-Atom. In besonderen Fällen kann es möglich sein, daß im Verlauf der erfindungsgemäßen Umsetzung dessen Konfiguration beeinflußt wird.

Die im erfindungsgemäßen Verfahren hergestellten Oxazolidin-2,4-dione sind bekannte Fungizide.

Beispiel 1

162 g (1 Mol) 3,4-Dichloranilin, 60 g (1 Mol) Harnstoff, 118 g (1 Mol) Milchsäureethylester wurden am Rückfluß erhitzt. Nach 4-5 Stunden betrug die Sumpftemperatur 230°C. Entstandenes Ethanol wurde laufend abdestilliert. Anschließend wurde heiß in 1500 ml Alkohol eingerührt und kalt abgesaugt. Es hatten sich 170 g = 65 % der Theorie an 3-(3',4'-Dichlorphenyl)-5-methyl-oxazolidin-2,4-dion Fp. 155°C gebildet.

Beispiel 2

93 g (1 Mol) Anilin, 60 g (1 Mol) Harnstoff, 118 g (1 Mol) Milchsäureethylester wurden am Rückfluß erhitzt und nachdem die Sumpftemperatur 200°C erreicht hatte, durch weitere Zugabe von Milchsäureester die Temperatur gehalten. Entstandener Alkohol wurde laufend abdestilliert. Nach 6 Stunden Reaktionszeit wurde in Alkohol eingerührt und abgesaugt.
Ausbeute: 160 g = 84 % der Theorie an
3-Phenyl-5-methyl-oxazolidin-2,4-dion
Fp. 143°C.

Beispiel 3

162 g (1 Mol) 3,5-Dichloranilin, 60 g (1 Mol) Harnstoff, 130 g (1 Mol) Vinylmilchsäuremethylester wurden langsam auf 220°C erhitzt und das entstandene Methanol laufend

abdestilliert. Nach 5 h wurde in 300 ml Methanol eingerührt und abgesaugt. Es hatten sich 187 g = 72 % der Theorie 3-(3,5-Dichlorphenyl)-5-methyl-5-vinyl-oxazolidin-2,4-dion (Fp. 108°C) gebildet.

Beispiel 4

24,3 g (0,15 Mol) 3,5-Dichloranilin, 9 g (0,15 Mol) Harnstoff, 19,5 g (0,15 Mol) 2-Methyl-1-hydroxycyclopropancarbonsäuremethylester wurden 3 h auf 220°C erhitzt. Entstandener Methylalkohol wurde abdestilliert. Mit Hilfe der Hochdruckflüssigkeitschromatographie wurde der Gehalt an

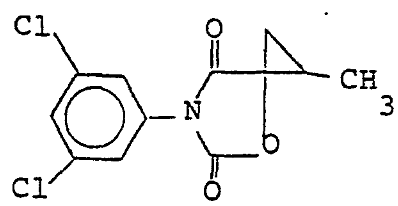

zu 68 % der Theorie bestimmt.

Beispiel 5

32,4 g (0,2 Mol) 3,4-Dichloranilin und 10,3 g (0,1 Mol) Biuret wurden auf 180°C erhitzt. Bei dieser Temperatur wurde langsam 23,6 g (0,2 Mol) Milchsäureethylester zugetropft und anschließend auf 230°C erhitzt. Entstandenes Ethanol wurde laufend abdestilliert. Nach insgesamt 2,5 Stunden Reaktionszeit wurde in 300 ml Ethanol eingerührt, abgekühlt und abgesaugt.

Es wurden 30,7 g = 59 % der Theorie an 3-(3',4'-Dichlorphenyl)-5-methyl-oxazolidin-2,4-dion vom Fp. = 155°C gewonnen.

Patentansprüche

1. Verfahren zur Herstellung von Oxazolidin-2,4-dionen der Formel I

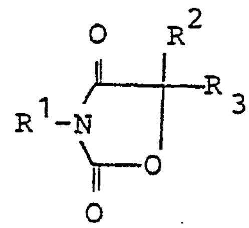

(I)

in der

$R^1$ für gegebenenfalls substituiertes Aryl steht,

$R^2$ und $R^3$ unabhängig voneinander für Wasserstoff, gegebenenfalls substituiertes Alkyl, Cycloalkyl, Vinyl stehen oder gemeinsam zusammen mit dem Kohlenstoffatom an den sie gebunden sind, einen gegebenenfalls substituierten Cycloalkylring bilden,

dadurch gekennzeichnet, daß man

2-Hydroxycarbonsäureester der Formel II

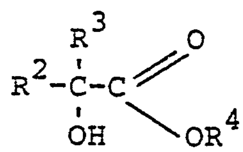

(II) .

worin

$R^2$ und $R^3$ die angegebene Bedeutung haben und

$R^4$ für Alkyl oder Cycloalkyl steht,

mit Harnstoff und/oder Ureten der Formel III,

$$H\text{-}(NH\overset{\displaystyle O}{\overset{\|}{C}})_n\text{-}NH_2 \qquad (III)$$

worin

n für eine ganze Zahl von 1 bis 4 steht,

und primären aromatischen Aminen der Formel IV,

$$R^1\text{-}NH_2 \qquad (IV)$$

worin

$R^1$ die oben angegebene Bedeutung hat,

bei Temperaturen von 100 bis 250°C umsetzt.

2. Verfahren nach Anspruch 1, dadurch gekennzeichnet, daß Verbindungen der Formel II eingesetzt werden, in der

$R^2$ und $R^3$ unabhängig voneinander für Wasserstoff, gegebenenfalls substituiertes $C_1$-$C_6$-Alkyl,

$C_3$-$C_6$-Cycloalkyl, $C_2$-$C_6$-Vinyl stehen, wobei $R^2$ und $R_3$ auch gemeinsam mit dem C-Atom an das sie gebunden sind einen $C_3$-$C_6$-Cycloalkylring bilden können und

$R^4$ für $C_1$-$C_{10}$-Alkyl und $C_3$-$C_6$-Cycloalkyl steht.

3. Verfahren nach Anspruch 1, dadurch gekennzeichnet, daß Verbindungen der Formel II eingesetzt werden, in der

$R^2$ und $R^3$ unabhängig voneinander für Wasserstoff, gegebenenfalls substituiertes Methyl, Ethyl, Propyl, Cyclopropyl, Cyclopentyl, Cyclohexyl, Vinyl stehen wobei $R^2$ und $R^3$ auch gemeinsam mit dem C-Atom an das sie gebunden sind, einen Cyclopropylring bilden können und

$R^4$ für $C_1$-$C_4$-Alkyl steht.

4. Verfahren nach Anspruch 1, dadurch gekennzeichnet, daß Verbindungen der Formel III eingesetzt werden, in der n für 1 oder 2 steht.

5. Verfahren nach Anspruch 1, dadurch gekennzeichnet, daß Verbindungen der Formel IV eingesetzt werden, in der $R^1$ für $C_1$-$C_{10}$-Aryl steht.

6. Verfahren nach Anspruch 1, dadurch gekennzeichnet, daß Verbindungen der Formel IV eingesetzt werden, in der $R^1$ für gegebenenfalls substituiertes Phenyl steht.

7. Verfahren nach Anspruch 1, dadurch gekennzeichnet, daß die Umsetzung in einem Verdünnungsmittel ausgeführt wird.

8. Verfahren nach Anspruch 1, dadurch gekennzeichnet, daß bei der Umsetzung biurethaltiger Harnstoff verwendet wird.

**0063299**

Nummer der Anmeldung

Europäisches Patentamt

# EUROPÄISCHER RECHERCHENBERICHT

EP 82 10 2885

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,Y | DE-A-1 811 843 (SUMITOMO) * Insgesamt und insbesondere Seite 3, Verfahren 2 und Seite 4, Verfahren 4 * --- | 1-8 | C 07 D 263/44<br>C 07 D 263/52 |
| Y | US-A-2 677 698 (A.J. DEUTSCHMAN) * Insgesamt * --- | 1-8 | |
| Y | US-A-2 806 051 (C.E. BROCKWAY) * Insgesamt * --- | 1-8 | |
| Y | DE-A-2 711 659 (BASF) * Insgesamt * --- | 1-8 | |
| D,Y | EP-A-0 007 415 (BASF) * Insgesamt * & DE - A - 2 827 414 --- | 1-8 | RECHERCHIERTE SACHGEBIETE (Int. Cl 3) |
| Y | CHEMICAL ABSTRACTS, Band 88, Nr. 21, 22. Mai 1978, Seite 598, Nr. 152500t, Columbus, Ohio, USA R.O. KOCHKANYAN et al.: "Novel method for the synthesis of azolidones" & KHIM. GETEROTSIKL. SOEDIN. 1978, (1), 87-89 * Zusammenfassung * ----- | 1-8 | C 07 D 263/00 |

Der vorliegende Recherchenbericht wurde für alle Patentansprüche erstellt.

| Recherchenort | Abschlußdatum der Recherche | Prüfer |
|---|---|---|
| DEN HAAG | 07-07-1982 | ALLARD M.S. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82